# EUROPEAN PATENT APPLICATION

(11) **EP 3 326 975 A1**
(43) Date of publication of application: **30.05.2018**
(21) Application number: 16382571.4
(22) Date of filing: 29.11.2016
(51) Int. Cl.: C01G 9/02, A01N 59/00, A61K 8/27, C09D 11/037, D21H 19/38

(54) **ZINC OXIDE MICROPARTICLES, PREPARATION METHOD, AND USE THEREOF**

(71) Applicant: Consejo Superior De Investigaciones Científicas, 28006 Madrid (ES)
(72) Inventor: FERNÁNDEZ LOZANO, José Francisco, E-28049 Madrid (ES); DE LUCAS GIL, Eva, E-28049 Madrid (ES); RUBIO MARCOS, Fernando, E-28049 Madrid (ES)
(74) Representative: ABG Patentes, S.L.

(57) **Abstract**

The present invention relates to biocide zinc oxide microparticles, preparation method and use thereof. More particularly, the present invention relates to zinc oxide microparticles comprising platelets, wherein the platelets have an edge length less between 30 nm and 200 nm, are stacked in direct contact with each other along the stacking c-axis by means of their [0001] or [0001] crystalline planes, and are rotated by a non-zero angle with respect to their adjacent platelets along said stacking c-axis, wherein said zinc oxide microparticles are characterised by a specific surface area which is less than or equal to 4 m²/g. The invention also relates to a method for preparing the zinc oxide microparticles of the invention, comprising the steps of i) adding urea to an aqueous zinc salt solution; ii) heating the solution resulting from step (i); iii) isolating the product resulting from step (ii); and iv) submitting the isolated product from step (iii) to an annealing treatment. The invention further relates to a cosmetic product, paint, ink, paper, cardboard, textile, food, agricultural, home care, air conditioning, animal care, personal and work hygiene, contact lens, chromatography material, medical equipment, dermatological, lacquer, coating and/or plastic product containing the zinc oxide microparticles of the invention and to a biocide composition comprising the zinc oxide microparticles as defined above. Finally, the invention further relates to the use of the biocide composition of the invention for the elimination, inhibition of the growth, or inhibition of progeny of microorganisms.

## Description

### FIELD OF THE INVENTION

The present invention relates to the field of zinc oxide particles and preparation method. More particularly, the present invention relates to the field of biocide zinc oxide microparticles.

### BACKGROUND

Zinc oxide (ZnO) materials find industrial applications in many technological fields. For example, ZnO materials show excellent optoelectronic properties (M. Bitenc et al., Cryst. Growth Des. 2010, 10, 830-837; Z. Hou et al., Nanoscale Res. Lett. 2012, 7, 507-513) because of their wide band gap energy of 3.37 eV and large exciton binding energy (K. Foe, et. al, Thin Solid Films, 2013, 534, 76-82; K. He, et al., CrystEngComm, 2014, 16, 3853-3856). Not only have they been widely studied as catalyst supports, such as in methanol synthesis or in decomposition from industrial and experimental processes (Phuruangrat, A. et al., Journal of Nanomaterials, 2014 (2014); US 20100284893) but they have also shown to excel in other applications such as in semiconductors in solar cells (P. Li et al., Mater. Chem. Phys. 2007, 106, 63-69; M. Klaumünzer et al., CrystEngComm, 2014, 16, 1502-1513) or for antimicrobial applications, of which antifungal (Patra P. et al., Langmuir, 2012, 28, 16966-16978; L. He et al., Microbiol. Res. 2011, 166, 207-215) and antibacterial (Talebian N. et al., J. Photochem. Photobiol. B: Biology, 2013, 120, 66-73; N. Padmavathy and R. Vijayaraghavan, Sci. Tech. Adv. Mater. 2008, 9) applications have been reported.

Additionally, ZnO is a particularly interesting oxide in material engineering because its self-assembly can be tampered with, allowing the formation of meso-, micro-, and nanostructures with precise control at the molecular level which in turn governs its structure, properties and function. By acting on the growth mechanisms of ZnO materials, different structures, shapes and more or less complex morphologies have been described. Examples of ZnO particles in 1D configuration are nanorods (Schlur L. et al., Chemical Communications, 2015, 51, 3367-3370), nanoneedles (Park W.I. et al., Adv Mater, 2002, 14, 1841-1843), nanowires (Yang P. et al., Advanced Functional Materials, 2002, 12, 323-331) and nanobelts (Pan Z.W. et al., Science, 2001, 291, 1947-1949). Most common 2D structures are nanosheets (Pan A. et al., J. Cryst. Growth, 2005, 282, 165-172) and nanopellets (Chiu W.S. et al., Chem. Eng. J., 2010, 158, 345-352). Regarding 3D structures, these include cauliflower (Lin L. et al., RSC Advances, 2015, 5, 25215-25221), snowflakes (Li C. et al., Nanoscale, 2010, 2, 2557-2560) and nanocages (Gao P.X. and Wang Z. L., JACS., 2003, 125, 11299-11305). Self-assembly of inorganic nano building blocks into one-dimensional, two-dimensional, and three-dimensional ordered nanostructures is appealing because the tuning of the way that building blocks arrange themselves provides a method to tune the final properties of the resulting material.

When it comes to the antimicrobial applications of ZnO materials, the vast majority of the background art refers solely to their antibacterial applications (Li, M. et al., Environ. Sci. Technol., 2011, 45, 1977-1983; Jones, N. et al., FEMS Microbiol. Lett., 2008, 279, 71-76) with little mention to their antifungal properties (Sharma, D., Thin Solid Films, 2010, 519, 1224-1229). In general, factors like particle concentration, size and specific surface area are sought by researches as decisive factors in the antimicrobial activity of ZnO. A clear common trend in the background art is that smaller, nanosized ZnO particles show increased antibacterial effectiveness. Although little is known regarding antifungal activity of ZnO materials, published research reveals a similar trend for ZnO antifungal activity: nanosized ZnO materials possess increased antifungal activity than micro- or meso- particles. This effect is usually associated with an increased specific surface area of smaller materials when compared to bulkier ones. In short, when it comes to antimicrobial activity of ZnO, the so far published consensus is that the smaller the better. However, a downside of nanosized ZnO materials is their toxicity: not only do nanosized ZnO particles become readily available to penetrate membrane cells or generate reactive oxygen species (ROS), but also ZnO materials are known to undergo leaching of toxic Zn²⁺ ions into the environment.

Therefore, a need exists for ZnO materials which show improved antimicrobial (both antibacterial and antifungal) activity without the downside of the above-mentioned toxicity phenomena.

### SUMMARY OF THE INVENTION

The authors of the present invention have grown low specific surface area ZnO microstructures of surprisingly increased antimicrobial activity with respect to commercial nanosized ZnO particles. The low specific surface area and micrometric size allow the particles of the invention to show very little toxicity associated to reduced levels of Zn²⁺ leaching and limited ROS production and showing high antimicrobial activity. In a broad sense, the particles of the invention comprise conical-shaped structures bound by their base, which are in turn comprised by platelets of ZnO. This configuration provides the particles of the invention with the advantageous properties of less toxic micro-sized ZnO particles and of increased antimicrobial activity, typical of nano-sized ZnO particles.

Accordingly, in a first aspect, the present invention is directed to zinc oxide microparticles comprising platelets, wherein the platelets,
- have an edge length between 30 and 200 nm, and
- are stacked in direct contact with each other along the stacking c-axis, by means of their [0001̅] or [0001] crystalline planes, and
- are rotated by a non-zero angle with respect to their adjacent platelets along said stacking c-axis,
wherein said zinc oxide microparticles are characterised by a specific surface area which is less than or equal to 4 m²/g.

A second aspect of the present invention relates to a method for preparing the zinc oxide microparticles as defined above, comprising the steps of i) adding urea to an aqueous zinc salt solution; ii) heating the solution resulting from step (i); iii) isolating the product resulting from step (ii); and iv) submitting the isolated product from step (iii) to an annealing treatment.

The inventors have surprisingly found that the microparticles of the present invention show intense antimicrobial activity and at the same time are less toxic than commercial nano- and micro-sized zinc oxide compositions.

Thus, a third aspect of the present invention relates to a cosmetic product, paint, ink, paper, cardboard, textile, food, agricultural, home care, air conditioning, animal care, personal and work hygiene, contact lens, chromatography material, medical equipment, dermatological, lacquer, coating and/or plastic product containing the zinc oxide microparticles as defined above.

In yet another aspect, the present invention is directed to a biocide composition comprising the zinc oxide microparticles as defined above and optionally a carrier.

Finally, another aspect of the present invention is directed to the use of the biocide composition as defined above for the elimination, inhibition of the growth, or inhibition of progeny of microorganisms.

### DESCRIPTION OF THE DRAWINGS

These and other characteristics and advantages of the invention will become clearly understood in view of the detailed description of the invention which becomes apparent from preferred embodiments, given just as an example and not being limited thereto, with reference to the drawings.

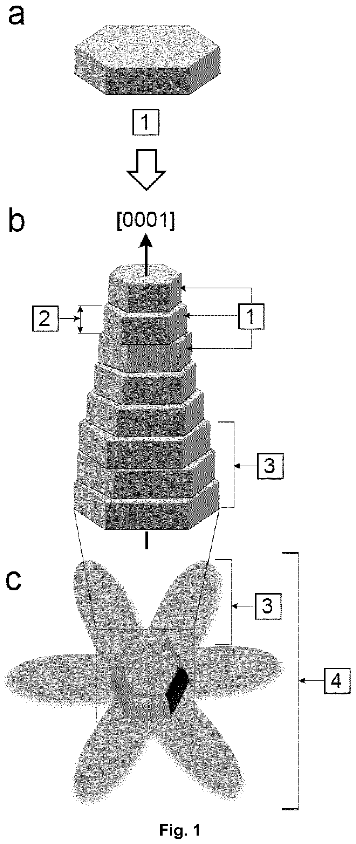

Figure 1: Representative growth mechanism of the microparticles of the present invention. Panel a shows a single ZnO platelet (1) and panel b displays the microstructure of the microparticles of the invention which comprises at least three ZnO platelets (3) stacked along the c-axis and has a conically-shaped morphology. The thickness of the short dimension of a single ZnO platelet is characterised by its edge length (2). Panel c shows a representation of a flower-like microparticle (4) that comprises conically-shaped platelets groups (3) bound by their base.

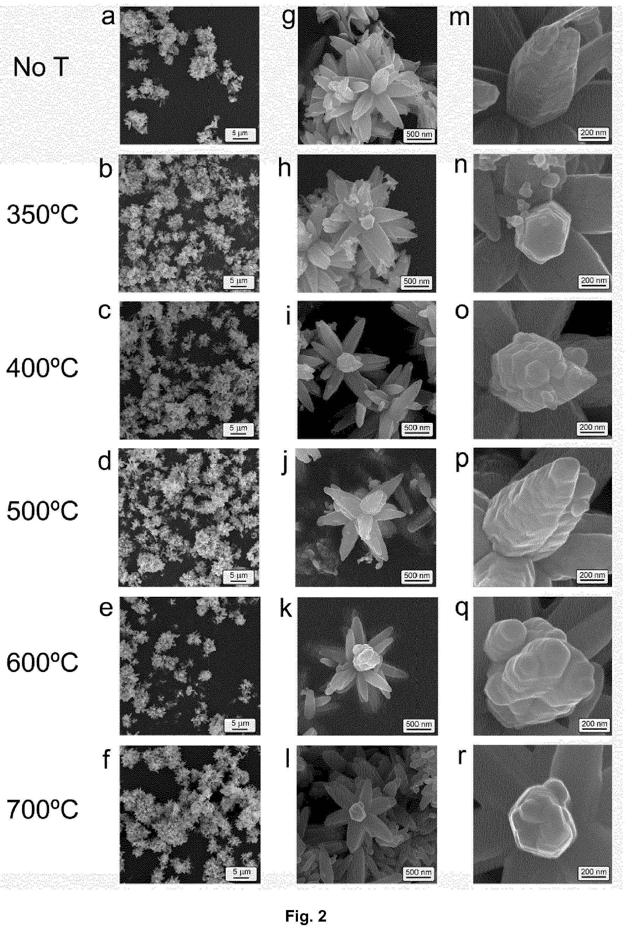

Figure 2: Low-resolution (panels a-f) and High-Resolution (panels g-l) scanning electron microscopy micrographs for morphological characterisation of the microparticles of examples 1 and 2, obtained before and after annealing thermal treatment for samples: A (no T, panels a, g and m); B (350 °C, panels b, h and n); C (400 °C, panels c, i and o); D (500 °C, panels d, j and p); E (600 °C, panels e, k and q) and sample F (700 °C, panels f, l and r). Panels m to r show the conically-shaped stacked platelet groups.

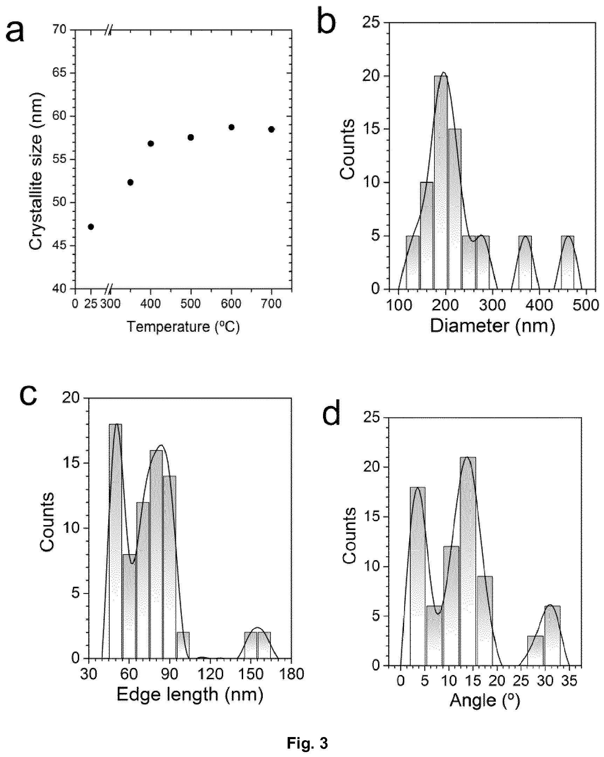

Figure 3: Crystallite size (panel a) and statistical study of microstructural parameter of microparticles in the Figure 2: platelet diameter (panel b), platelet thickness defined as edge length (panel c) and rotation angle between platelets separated by a domain wall (panel d).

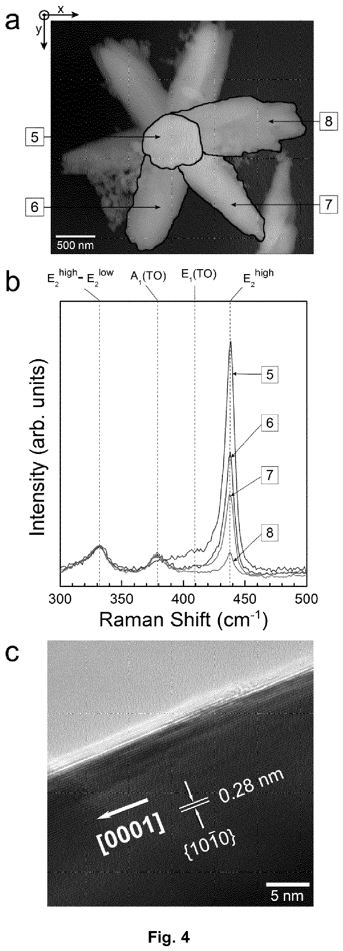

Figure 4: Scanning electron micrograph of a flower-like microparticle of the invention (panel a) thermally treated at 500°C (sample D) in which different conically-shaped platelet groups (5) (6) (7) and (8) are signalled respect to the reference x and y axis. Panel b shows the Raman spectra of each conically-shaped platelet group that reveals the crystal orientation related the intensity of the E₂^{high} Raman mode of ZnO. The 532 nm laser employed in the Raman experiment was a polarized laser along the x axis. Panel c shows a transmission electron micrograph of a microparticle thermally treated at 500°C (sample D) showing the [0001] crystallographic direction parallel to the edge length side of the platelet and therefore along the stacking direction of ZnO platelets.

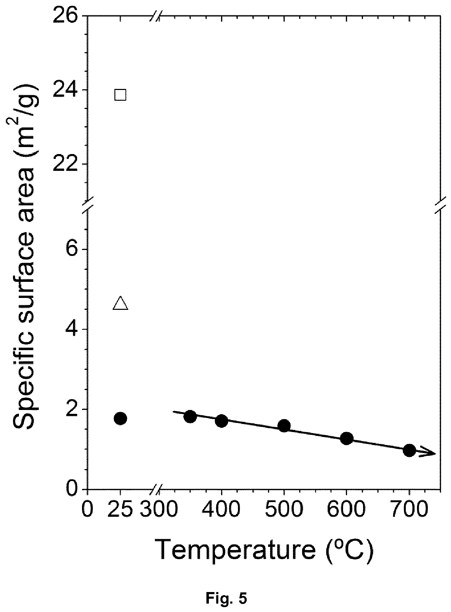

Figure 5: Specific surface area (SSA) values of two commercial samples, nanoZnO (square unfilled) and microZnO (triangle unfilled) as well as the particles of the invention (circle filled), as a function of the annealing temperature.

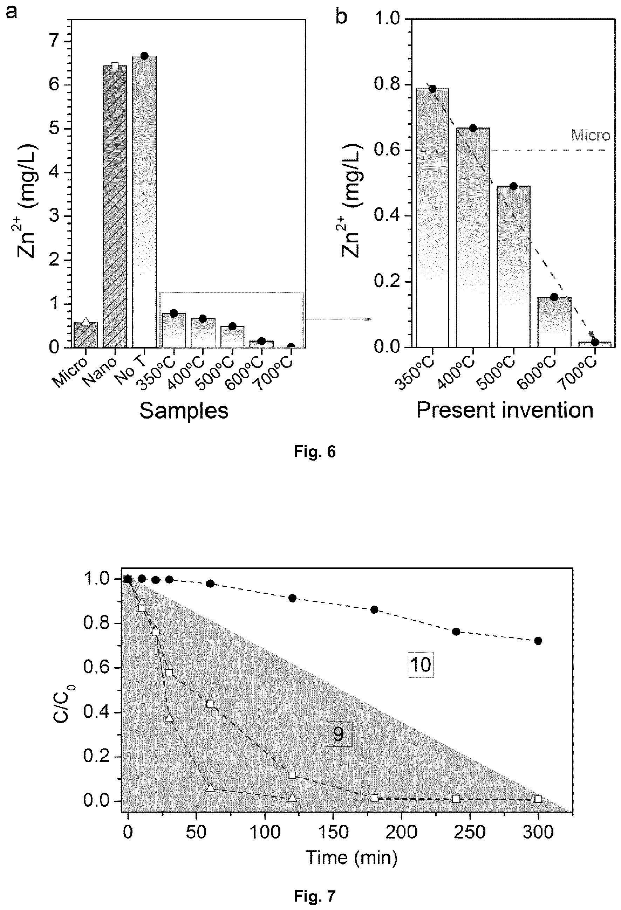

Figure 6: Zn²⁺ leaching assay in peptone water of example 4 (panel a). Data was obtained by means of Inductively Coupled Plasma Atomic Emission Spectroscopy (ICP-AES) which yielded the Zn²⁺ concentration in peptone water for each of the tested samples: commercial samples microZnO and nanoZnO, and samples A to F (examples 1 and 2). A graphical representation wherein the ordinate axis (corresponding to the Zn²⁺ concentration) is limited to a value of 1 mg/L is shown in panel b.
Figure 7: Photocatalytic degradation of methyl orange (MO) exposed to UV-light irradiation in the presence of commercial samples microZnO (triangle unfilled) and nanoZnO (square unfilled) as well as the particles exemplary of the present invention (sample D, circle filled). The graph area is divided in two regions according to the photocatalytic degradation capacity. The grey zone (9) corresponds to a region in which ZnO particles produce large photocatalytic degradation and the white zone (10) indicates that ZnO particles produce low photocatalytic degradation.

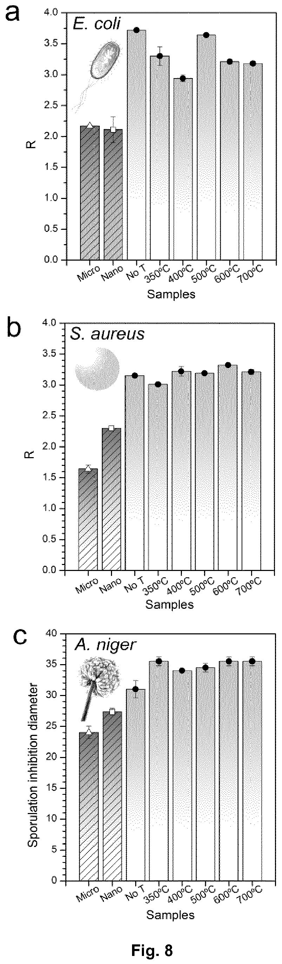

Figure 8: Antimicrobial activity of the particles of the present invention represented by samples A to F, compared with commercial references (microZnO and nanoZnO). Panels a and b represent the antibacterial activity (R) against *E. coli* and S. *aureus.* Panel c shows the antifungal activity of said samples against *A. niger.*

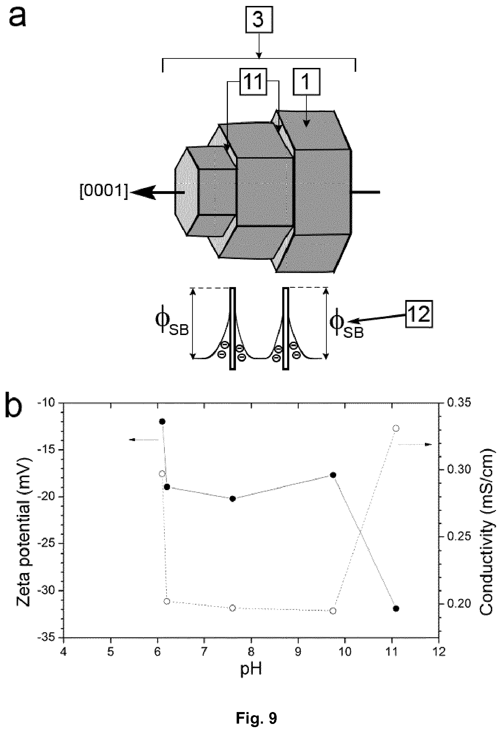

Figure 9: Panel a shows a three platelet (3) unit of the present invention microparticles in which each platelet is stacked along [0001] direction. Adjacent platelets form a domain wall (11) interface in which adjacent platelets are rotated along the [0001] direction. The charge accumulation generated at the domain walls forms a potential barrier called Schottky barrier (12). Panel b displays the zeta potential and the conductivity values of ZnO microparticles of the present invention (sample D) measured in a water suspension at different values of pH.

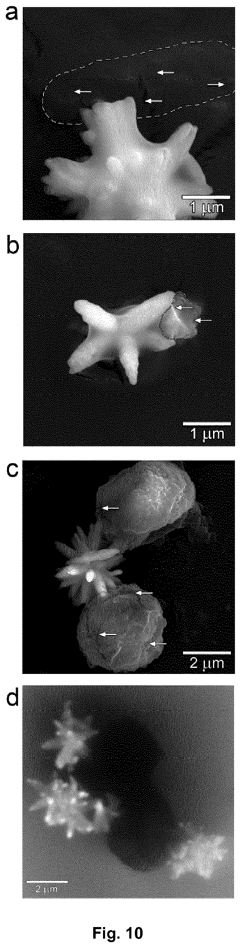

Figure 10: Antimicrobial micrographs of ZnO microparticles of the present invention (sample D) attacking bacteria *E. coli* (a), *S*. *aureus* (b) and fungus *A. niger* (c) by electrostatic action. Panel d shows the cathodoluminiscence of sample D against A. *niger.* The dotted line in the Figure 10(a) delimits the *E. coli* bacterium and in Figure 10(b) delineates the *S*. *aureus* bacterium.

### DETAILED DESCRIPTION OF THE INVENTION

Unless defined otherwise, all technical and scientific terms and expressions used herein have the same meaning as commonly understood to one of ordinary skill in the art to which this disclosure belongs.

A first aspect of the invention is directed to zinc oxide microparticles comprising platelets, wherein the platelets,
- have an edge length between 30 and 200 nm, and
- are stacked in direct contact with each other along the stacking c-axis, by means of their [0001̅] or [0001] crystalline planes, and
- are rotated by a non-zero angle with respect to their adjacent platelets along said stacking c-axis;
wherein said zinc oxide microparticles are characterised by a specific surface area which is less than or equal to 4 m²/g.

The specific surface area (SSA) of a solid is to be understood as the total surface area of said solid per unit of mass, and it is expressed in m²/g. Under this definition, it becomes clear to the skilled person that a flat surface has a less SSA value than an accordion-like surface. In this regard, the SSA values for solids can be easily measured by means of Brunauer-Emmett-Teller (BET) isotherm adsorption curves.

A nanomaterial is considered when 50 % or more of the particles in their number size distribution possesses one or more external dimensions in the size range between 1 nm to 100 nm. The minimum value of the specific surface area (SSA) for different particle morphologies (sphere, fibre and plate) having one dimension as 100 nm was calculated for referencing purposes. To calculate SSA values it is necessary to determine the particle volume and the area for each morphology as well as the number of particles per gram. To express the data as a mass function, instead of using the number of particles, the density 5.61 g /cm³ value of ZnO material was used. The results obtained for sphere, fibre and plate morphologies were 10.7 m²/g, 7.5 m²/g and 4.3 m²/g, respectively. The minimum SSA calculations allow categorizing the particles into two groups: nanoscale group (SSA more than 10.7 m²/g) and microscale group (SSA less than 4.3 m²/g). The potential toxicity ranges of the particles are defined accordingly to the minimum SSA as unsafe (more than 10.7 m²/g), safe limit (between 10.7 and 4.3 m²/g) and safe (less than 4.3 m²/g).

The widely used term "microparticle", is to be interpreted in the context of the present invention as any particle having at least one micrometric dimension, *i.e.,* smaller than 100 µm but greater than 100 nm.

The term "stack" is to be interpreted as an orderly pile of, in the present context, platelets.

In the context of the present invention, the term "platelet" is to be associated with any ZnO particle characterised by being of plate-like shape (1 in Figure 1) or a prism with hexagonal type base.

The term "edge length" (2 in Figure 1) when used to refer to the platelet thickness refers to the short edge between the two closest vertices of each polygon base of the crystalline particle. The term "side length" refers to the large edge of the two closest vertices of the polygon base. The term "diameter" or "equivalent diameter" of the platelet refers to the distance between two farthest vertices of the polygon base. For the sake of exemplifying the latter, if the polygon in question is a hexagonal prism, then the edge length would be the height of said prism, *i.e.,* the length of one of the six edges formed between two closest vertices of each hexagonal base that form the hexagonal prism. The term "vertex" refers to the union of two adjacent edges. The term rotation angle between two adjacent platelets refers to the angle formed by the ray which goes from the center of a first platelet to one vertex of the first platelet and the ray which goes from the center of a second platelet (adjacent to the first platelet) to one vertex of the second platelet (and equivalent to the vertex considered for the first platelet). The rotation angle occurs along the c-axis of the ZnO platelets.

In a particular embodiment of the invention, the platelets are single crystals. In another particular embodiment of the invention, each platelet creates a single domain.

In the context of the present invention, the term "domain" refers to a region within a ZnO crystal in which the crystal is homogeneous in a crystallographic direction.

Moreover, the zinc oxide microparticles, as defined above, comprise platelets having an edge length between 30 and 200 nm.

In a particular embodiment of the invention, the platelets are characterised by an edge length between 40 and 180 nm.

In another particular embodiment of the invention, the platelets are characterised by a diameter between 100 and 500 nm.

The term "crystallite size" is to be interpreted as the coherence length of the crystal structure and it is determined by X Ray Diffraction.

In a particular embodiment of the invention, the ZnO microparticles are characterised by a crystallite size between 10 nm and 100 nm, preferably between 40 and 70 nm.

The zinc oxide microparticles of the invention comprise stacked platelets along the hexagonal c-axis.

In a particular embodiment of the invention, the stacked platelets form domain walls or Schottky-like barriers.

The inventors have surprisingly found that the microparticles of the invention are formed by platelets which are stacked in direct contact with each other along the stacking c-axis by means of their [0001̅] or [0001] crystalline planes, *i.e.,* their polar faces, and are rotated by a non-zero angle with respect to their adjacent platelets along said stacking c-axis. The rotation angle formed between two adjacent platelets joined by domain walls is a result of the conically-shaped stacked structure in which the adjacent platelets possess different size and therefore the six vertices are un-aligned as the platelet diameter decreases along the growth axis, the c-axis of the ZnO crystalline structure.

In a particular embodiment of the invention, the non-zero angle is an angle comprised between 1° and 40°.

Without wishing to be bound by any particular theory, it is believed that it is the direct contact between adjacent platelets at an angle different from 0° along the stacking c-axis what gives rise to the generation of domain walls at each interplatelet layer, by accumulating electric charge and generating an electric potential distributed throughout the structure. A likely explanation of the assembly mechanism and electric phenomena behind the particles of the invention follows.

At ambient conditions, zinc oxide (ZnO) crystallizes mainly in the most stable hexagonal *wurtzite* form, a polar crystal which is characterized by a positively charged Zn-polar surface ([0001] crystalline plane) and a negatively charged O-polar surface ([0001̅] crystalline plane) in addition to the six non-polar faces. The [0001] or [0001̅] crystalline planes are the c-axis crystallographic direction of the ZnO structure. In terms of the ZnO crystalline molecular structure, the polar surfaces are associated to Zn and O atoms placed in terminal positions. Schematically, these positively and negatively charged surfaces are believed to play an important role in the mechanism behind the formation of stacked platelets along the c-axis: it is the electrostatic attraction between said polar surfaces what pulls them together and allows the stacking of the ZnO nanoplatelets (Wang S. and Xu A., CrystEngComm, 2013, 15, 376).

Once the ZnO microparticles comprising stacked platelets are formed, a thermal treatment or thermal annealing compresses said platelets against their neighbouring (adjacent) platelets, giving rise to platelets which are in direct contact with their adjacent platelets. As a consequence of the thermal treatment, the adjacent platelets form a domain wall at the interface. Moreover, the thermal treatment improves the crystallinity of the structure that results in a crystallite size between 10 nm and 100 nm, preferably between 40 and 70 nm. Larger crystallites sizes of stacked platelets results in a greater immobilization of zinc cations, *i.e.* the thermal treatment reduces the Zn²⁺ release as demonstrated in leaching experiments.

In addition, the stacking of the ZnO platelets along the c-axis occurs at a non-zero rotation angle, i.e., an angle of rotation which is >0°, with respect to their adjacent platelets meaning that each platelet has a degree of rotation along the c-axis with regards to its two adjacent platelets. It is believed that, in these conditions, each platelet is a single domain, and it is the boundaries formed by each platelet what gives rise to a finite interfacial phenomena and the appearance of domain walls (11) (Figure 9). As it can be seen in the Figure 9(a), the domain walls lead to a generation of Schottky-like barriers, where a negative charge is accumulated at the depletion of the ZnO conduction band. Thus, these Schottky barriers entail the generation of an electric potential. The Schottky barriers can be asymmetric due to the relative rotation, size, among others, of each platelet. Moreover, the presence of multiple stacked platelets gives rise to the appearance of multidomains which could be regarded as a particle or a structure that comprises more than, or at least, two domains.

In the context of the present invention, the term "domain wall" refers to an interface separating crystallographic domains wherein, as defined above, each domain is a region within a ZnO crystal in which the crystal is homogeneous in a crystallographic direction. Regarding the preferential growth axis, the platelets are oriented along the c-axis giving rise to a conically-shaped stacked platelet structure. The domain wall occurs in the plane perpendicular to the principal axis of the ZnO crystal system, the c-axis and the crystal symmetry is broken by the rotation of the adjacent platelets along c-axis.

The zinc oxide microparticles of the present invention are characterised by a specific surface area which is less than or equal to 4 m²/g.

In a preferred embodiment of the invention, the zinc oxide microparticles as defined above are characterised by a specific surface area which is less than or equal to 2 m²/g, more preferably less than or equal to 1.5 m²/g.

It is believed in the context of the present invention that one of the reasons behind ZnO nanoparticle toxicity refers to the particle size being of nanometric dimensions. Similarly, it is also believed in the context of the present invention that ZnO nanoparticle toxicity is also related to their specific surface area (SSA). Taking SSA as a criterion to evaluate ZnO toxicity, it was determined that the particles of the invention are far safer than commercial spherical nanosized ZnO (nanoZnO, *Evonik*) and plate microsized (microZnO, *Asturiana de Zinc*) commercial samples.

The zinc oxide microparticles of the invention may be microparticles wherein the stacked platelets form a conical-shaped structure, and wherein at least two of said conical-shaped structures are bound by the base of each cone. This structure can be schematically represented by panel c of Figure 1.

In a particular embodiment, each conical-shaped structure of the microparticles of the invention is formed by at least three platelets. For example, a conical-shaped structure having a length of 500 nm could be formed by around 10 stacked platelets having an edge length of around 50 nm.

In a particular embodiment, the conical-shaped structures of the invention are characterised by a diameter between 0.1 and 0.5 µm, more preferably between 0.1 and 0.3 µm.

In a preferred embodiment of the invention, the zinc oxide microparticles of the invention are characterised by being flower-like shaped microparticle structures. The SEM images in Figure 2 show non-limiting representatives of these flower-like microparticles of the invention. In this way, the term "flower-like" refers to a shape that resembles a flower, or similar figures such as a bouquet, a tree, a star, a cactus, among others.

In a particular embodiment, the flower-like microparticles of the invention are characterised by a diameter comprised between 0.1 and 20 µm, more preferably between 1 and 10 µm.

Another aspect of the invention relates to a method for preparing the zinc oxide microparticles as defined above, comprising the steps of i) adding urea to an aqueous zinc salt solution, ii) heating the solution resulting from step (i), iii) isolating the product resulting from step (ii), and iv) submitting the isolated product from step (iii) to an annealing treatment.

In another preferred embodiment, the method for preparing the zinc oxide microparticles as defined above is a method wherein the zinc salt solution of step (i) is at a concentration between 1 and 10 M, more preferably between 4 and 6 M, even more preferably 5 M.

In yet another preferred embodiment, the method for preparing the zinc oxide microparticles as defined above is a method wherein the solution in step (ii) is heated to a temperature between 80 °C and 140 °C for a period of time between 1 and 3 hours.

In a more preferred embodiment of the invention, the solution in step (ii) is heated to a temperature between 100 °C and 120 °C.

In yet another particular embodiment of the invention, the solution in step (ii) is heated for 2 hours.

The term "isolating" refers to the any process capable of producing the main reaction product of step (ii) deprived of most liquid material. For example, isolating the product may refer to the process of extraction, centrifugation, filtration, evaporation, crystallization, and other processes known in the art.

In a preferred embodiment of the invention, the method for preparing the zinc oxide microparticles as defined above is a method wherein step (iii) comprises cooling down the solution resulting from step (ii) to room temperature, and adding a hot aqueous solvent to obtain a precipitated product; and isolating the precipitated product. Once the precipitated product is isolated, it can be optionally washed with a solvent selected from polar or non-polar solvents as the one from the group of water, ethanol, methanol, isopropanol, acetone, methyl acetate, ethyl acetate, dimethylformamide, acetonitrile, pentane, hexane, toluene, turpentine, tetrachloroethylene.

In a particular embodiment, the hot aqueous solvent of step (iii) is water.

In another preferred embodiment, the method for preparing the zinc oxide microparticles of the invention is a method wherein the annealing treatment (step iv) is performed at a temperature between 350 °C and 700 °C.

In yet another preferred embodiment, said annealing treatment of step (iv) is performed for a period of time between 1 and 24 hours.

The term "annealing" is widely employed in metallurgy and materials science, and refers to a heat treatment that alters the physical and sometimes chemical properties of a material to increase its ductility and reduce its hardness, making it more workable. It involves heating a material to above its recrystallization temperature, maintaining a suitable temperature, and then cooling.

The particles of the invention have been found to exert a biocidal effect when in contact with microorganisms. Therefore, another aspect of the invention refers to a biocide composition comprising the zinc oxide microparticles as defined above and optionally a carrier.

While any suitable carrier known to those of ordinary skill in the art may be employed in the biocide compositions of this invention, the type of carrier will vary depending on the type of application. Biocide compositions of the present invention may be formulated comprising a liquid carrier wherein examples of liquid carriers include, but are not limited to, a liquid, such as water, saline, alcohol, a fat, a wax or a buffer, or a solid carrier wherein examples of solid carriers include, but are not limited to, a solid such as a polymer, mannitol, lactose, starch, magnesium stearate, sodium saccharine, talcum, cellulose, glucose, sucrose, magnesium carbonate which can, both liquid and/or solid carriers, carry a dispersion, suspension, or solution. Other suitable carriers include emulsions, paste, ointments, gels, creams, lotions, powders, oils, pencils, deodorants-creams, gels, lotions, emulsions, deodorant sticks, roll-ons, sprays, pump sprays or lacquers.

The inventors have found that the microparticles of the invention have excellent antimicrobial properties, being suitable as biocidal agents for any kind of microscopic uni- or multicellular living organisms such as bacteria, archaea, protozoa, fungi and algae. Additionally, the microparticles of the invention act on living microorganisms by a mechanism which, surprisingly, is not based on Zn²⁺ leaching or on the generation of reactive oxygen species (ROS). This is evidenced by Figures 6 and 7, as well as examples 4 and 5, and is a paradigm-shifting finding that goes against the teachings of the prior art.

The term "biocide", employed in the current context when referring to the microparticles of the invention or a composition and/or material comprising said microparticles, is to be interpreted as the quality inherent to the microparticles of the invention to destroy, deter, render harmless, inhibit growth, exert a controlling effect or induce apoptosis on any microorganism. In the context of the present invention, the term "dose" refers to the required concentration of the ZnO microparticles of the present invention to produce such biocide effect. The term "biocide" is also to be interpreted as having a similar meaning as a disinfectant, pesticide or preservative. According to the present disclosure, it is apparent to the skilled person that the biocidal ZnO microparticles as defined above can be incorporated in any material suitable for being put in close contact with any surface that requires being disinfected.

Therefore, in another aspect of the invention, the invention is directed to a cosmetic product, paint, ink, paper, cardboard, textile, food, agricultural, home care, air conditioning, animal care, personal and work hygiene, contact lens, chromatography material, medical equipment, dermatological, lacquer, coating and/or plastic product containing the zinc oxide microparticles of the invention.

In a particular embodiment, the invention is directed to a product selected from paints, inks, agricultural, home care products, air conditioning, animal care, products for work hygiene, chromatography materials, medical equipment, lacquers, coatings and/or plastics containing the zinc oxide microparticles of the invention.

In another particular embodiment, the invention is directed to cosmetic products, food, agricultural, animal care, products for personal hygiene, contact lenses, medical equipment and/or dermatological products containing the zinc oxide microparticles of the invention.

In yet another particular embodiment of the present invention, the microparticles as defined above can be used in the manufacture of materials which are in close contact with human, animal or plant organisms. This application is possible due to the low particle dose required by the present invention in addition to the advantageous properties of the particles of the invention: low generation of reactive oxygen species (ROS) and low release of Zn²⁺ cations.

In yet another aspect, the invention is directed to the use of a biocide composition for the elimination, inhibition of the growth, or inhibition of progeny of microorganisms.

In a preferred embodiment of the invention, the invention is directed to the use of a biocide composition wherein the microorganisms are selected from bacteria and fungi.

In the context of the present invention, the term "bacterium" (plural "bacteria") refers to both gram-negative and gram-positive prokaryotic microorganisms, typically a few micrometres in length and having a large number of shapes, ranging from spheres to rods and spirals. Non-limitative examples of bacteria suitable for the use of the biocide composition of the present invention are Escherichia coli (E. coli), Staphylococcus aureus (S. aureus) and Aspergillus niger (A. niger).

In the context of the present invention, the term "fungus" (plural "fungi" or "funguses") refers to eukaryotic microorganisms that include unicellular microorganisms such as yeasts and molds, as well as multicellular fungi that produce familiar fruiting forms known as mushrooms.

Non-limitative examples of fungi suitable for the use of the biocide composition of the present invention are brown-rot (Poria Sp.), white-rot (Polyporous Sp.), wood decay fungi (Poria placenta) and mold.

### EXAMPLES

The present invention will now be described by way of examples which serve to illustrate the construction and testing of illustrative embodiments. However, it is understood that the present invention is not limited in any way to the examples below.

### Example 1. Synthesis of an exemplary microparticle of the invention

All chemicals were directly used without further purification. To start the procedure, 3.6 mol urea (CO(NH₂)₂) was added to a 5 M zinc nitrate hexahydrate (Zn(NO₃)₂·6H₂O) solution under stirring at room temperature. The reaction was heated in an oil bath at 100-120°C and stirred at 300 rpm for 2 h. After naturally cooling down to room temperature, hot water was added to obtain a white suspension. Subsequently, the precipitate was isolated by filtration and washed with water and ethanol to remove impurities. The white powder product was dried at 80 °C for 24 h to yield an exemplary sample A (Figure 2, panels a, g and m).

### Example 2. Thermal annealing treatment of an exemplary microparticle of the invention.

The dried product obtained in example 1 (sample A) was further submitted to an annealing treatment at temperatures between 350°C and 700°C, giving rise to samples B (350 °C), C (400 °C), D (500 °C), E (600 °C) and F (700 °C). The annealing treatment consisted in placing a ZnO sample obtained from a method such as the one in example 1, in an electric furnace (Nabertherm) for 1 to 24 hours under air atmosphere. Figure 2 shows SEM images of the exemplary microparticles of samples B to F.

Respect to the characterization of present invention, a statistical study of the main morphological parameters has been performed (Figure 3). Figure 3(a) shows that thermal treatment has an important role in the crystallite size of the present invention. As can be seen in Figure 3(b), the platelet diameter is found to be between 100 and 500 nm, where the average value is 231 nm. Figure 3(c) displays the edge length histogram between 30 and 180 nm and which average is 76 nm. The non-zero angle histogram characteristic of this invention is represented in Figure 3(d). The average angle is 13° but may vary between >0° to 35°.

### Example 2. Raman characterisation of the microparticles of the invention

Panel a of Figure 4 shows the scanning electron micrograph of an exemplary single microparticle of the invention (sample D), comprising differently conical-shaped groups arranged in different spatial directions. For the sake of simplicity, a reference x-y axis was drawn in the micrograph. Each conical-shape structure comprised a manifold of stacked ZnO platelets. This exemplary microparticle is characterized by a flower-like shape. The same microparticle was observed under the optical microscope of the Raman Confocal Microscope and the x-y axis reference was aligned accordingly. A 534 nm polarized laser along the x-axis was used for Raman spectroscopy. As can be seen in panel b (Fig. 4), the intensity of the E₂^{high} signal varies with the orientation of each stacked conical-shape group that forms the flower-shaped microparticle. In the case of ZnO, the E₂^{high} Raman mode possesses a higher Raman intensity when polarized light is aligned perpendicularly to the crystallographic plane [0001]. The Raman spectra depicted in panel b of Figure 4 are identified with the corresponding conical-shape platelet groups shown in panel a of Figure 4. The most intense Raman peak (5) corresponds with the vertically oriented platelet stacking. However, the conical-shaped groups oriented in the x-y plane (6, 7, 8) shows less intense E₂^{high} Raman peaks according to the projection in the y-axis that it is also perpendicular to the polarized light direction. The above results support that the platelets are stacked along the [0001] crystallographic direction. Transmission electron microscopy (TEM) confirms this result (panel c of Figure 4). TEM micrograph shows the alignment of the [0001] crystalline structure parallel to the edge length of a ZnO platelet. In this case, the crystalline plane observed in the TEM micrograph corresponds to the surface [101̅0], and the preferential growth direction of the stacked platelet group is [0001], i.e. the c-axis.

### Example 3. Determination of the specific surface area for the particles of the invention.

The SSA of the exemplary ZnO flower-shaped microparticles is determined by the Brunauer-Emmett-Teller (BET) method. For the sake of comparison, two different sized commercial products are used in this study: microZnO (Asturiana de Zinc) and nanoZnO (Evonik) references. The data is represented in Figure 5. The SSA value corresponding to the nanoZnO reference is located in the unsafe range (more than 10.7 m²/g), corresponding to the nanoscale behaviour. The SSA value for the microZnO commercial reference is found to fall in the safe limit (between 10.7 and 4.3 m²/g), close to the microscale behaviour. With respect to the flower-like ZnO structures samples A to F (see Examples 1 and 2), the experimentally determined SSA values are lower than commercial microZnO. These samples fall within the microscale behaviour (less than 4.3 m²/g), and therefore their potential toxicity is established in the safe range. In addition, the evolution of the SSA values with the annealing treatment (arrow in Figure 5) reveals that for higher temperature treatments, the specific surface area decreases (see table below). Thus, it is suggested that by increasing the annealing temperature, the stacked platelets of the particle of the invention can be increasingly compacted against each other, blocking some pores which are possibly created during synthesis. Hence, the annealing temperature plays an important role in the compaction process of stacked platelets, which allows tuning the SSA values in the particles of the invention, reaching values below 1 m²/g (sample F).

| **Sample** | **SSA (m²/g)** |
|---|---|
| MicroZnO | 4.61 |
| NanoZnO | 23.87 |
| A | 1.77 |
| B | 1.82 |
| C | 1.71 |
| D | 1.59 |
| E | 1.27 |
| F | 0.97 |

### Example 4. Leaching of cationic zinc for ZnO samples in peptone water.

The inventors have surprisingly found that the particles of the invention do not leach cationic zinc into the environment when compared to commercial nanoZn samples and that the amount of leached Zn²⁺ ions by the particles of the invention is falls well below the values of commercial microZn samples. In this regard, a leaching assay of Zn²⁺ was performed for samples A to F as well as for the comparative commercial samples microZnO and nanoZnO in peptone water. The assay consisted in preparing a suspension of 10g/L of ZnO (commercial samples and samples of invention) in 25 mL of peptone water (PW) for 24 hours at 37°C and collect the obtained colorless solution obtained. Figure 6 shows the leaching assay results by representing the ICP-AES measured Zn²⁺ concentration in peptone water for each of the samples. The concentration of Zn²⁺ release for the flower-like ZnO particles of the invention is compared with commercial references (microZnO and nanoZnO). Figure 5a shows that the non-thermal treated particle (sample A) and nanoZnO sample are responsible for higher leaching of Zn²⁺ in solution, whereas the microZnO commercial sample and flower-like ZnO samples B to F values are well below 1 mg/L. In addition, Figure 6b displays in detail the relationship between the amount of leached Zn²⁺ and annealing treatments. The leached Zn²⁺ decreases with increasing temperature, possibly due to the stabilization and compaction of the ZnO platelet stacked structure. At 700°C (sample F) the measured released Zn²⁺ is equal to values as low as 0.02 mg/L. Therefore, the use of thermal treatment allows controlling Zn²⁺ release.

### Example 5. Reactive Oxygen Species production.

The inventors have surprisingly found that the microparticles of the invention do not engage into significant production of reactive oxygen species (ROS) when submitted to light, contrary to commercial nanoZn and microZn samples. In this regard, the photocatalytic performance of the particles of the invention (sample D in example 2) as well as the comparative commercial samples were evaluated by studying the photodegradation of the organic dye methyl orange (MO) under UV-light. The results of the MO photodegradation in presence of an exemplary microparticle of the invention, commercial nanoZn and commercial microZn samples as a function of time are graphically shown in Figure 7. As can be seen, the microZnO commercial sample (triangle unfilled) induces the complete degradation of MO at 2h and the nanoZnO commercial sample (square unfilled) photodegrades MO in 3h. Surprisingly, the flower-like ZnO microparticles (sample D, circle filled) are only capable of inducing the photodegradation of 30% of the MO dye and only after 5 h of radiation. This result clearly indicates two zones in the Figure 7, one where the generation of ROS predominates (9) (Figure 7) and another where ROS are not produced (10) (Figure 7). Therefore, the ROS generation in commercial microZnO and nanoZnO, which are located in the grey region (9) (Figure 7), is much higher than the flower-like ZnO microparticles (sample D). Besides, this behaviour of less ROS production extends to other samples such as B or F as can be seen in the table below.

| **Time (min)** | **MicroZnO (C/C₀)** | **NanoZnO (C/C₀)** | **Sample D (C/C₀)** | **Sample B (C/C₀)** | **Sample F (C/C₀)** |
|---|---|---|---|---|---|
| 0 | 1.000 | 1.000 | 1.000 | 1.000 | 1.000 |
| 10 | 0.892 | 0.868 | 1.000 | 1.000 | 1.000 |
| 20 | 0.766 | 0.760 | 0.996 | 0.992 | 1.000 |
| 30 | 0.373 | 0.578 | 0.998 | 0.989 | 1.000 |
| 60 | 0.056 | 0.438 | 0.979 | 0.958 | 0.988 |
| 120 | 0.011 | 0.116 | 0.915 | 0.904 | 0.905 |
| 180 | 0.009 | 0.015 | 0.862 | 0.832 | 0.802 |
| 240 | 0.007 | 0.010 | 0.764 | 0.766 | 0.708 |
| 300 | 0.005 | 0.009 | 0.722 | 0.710 | 0.615 |

### Example 6. Biocide activity

The broad biocide activity of the ZnO microparticles of the invention, exemplified by samples A to F (example 1 and 2) was studied against three microorganisms: two bacteria, being *Escherichia coli* (CECT 516) and *Staphylococcus aureus* (CECT 240) and one fungus, *A. niger* (CECT 2807). Regarding the two bacteria, *E. coli* is a rodshaped Gram-negative bacterium while *S*. *aureus* is a round-shaped Gram-positive bacterium. The antibacterial activity study was performed by the macrodilution method following the National Committee of Clinical Laboratory Standards (NCCLS) with some slight modifications. Figure 8 (panels a and b) shows the antibacterial activity values (denoted as R) calculated from colony forming units (CFU) of bacteria after adding 3 ppm of the ZnO sample under study (commercial and samples A-F) and subsequent incubation for 24 h at 37 °C. As can be seen from panels a and b of figure 8, commercial microZnO and nanoZnO induces a decrease in the bacterial population, reaching R values of about 2 for *E.coli* and of about 1.5-2.3 for *S*. *aureus.* In contrast, the presence of the exemplary particles of samples A-F show a dramatic decrease in the bacterial population reaching R values of about 3-3.7 for *E. coli* and about 3-3.3 for *S*. *aureus.* These values translate into the particles of the present invention being capable of enhancing the antibacterial activity of traditional ZnO by about 70% for Gram-negative and 50% for Gram-positive bacteria. Turning to fungus growth, the study was performed recurring to the Kirby-Bauer method. Fungus *A. niger* (CECT 2807) is cultivated on petri dishes containing the particles of present invention, exemplified by samples A to F, thermally treated and incubated at 37 °C for 3 days. For comparison purposes, different sized commercial references were used as before. Figure 7 (panel c) shows the reduction of fungal growth in presence of samples A to F as well as microZnO and nanoZnO references. The results showed that the particles of the present invention increase the antifungal activity by about 42% with respect to microZnO and 26% with respect to nanoZnO. These results again showed that the particles of the present invention significantly inhibit the growth of *A. niger* and were more effective than ZnO commercial products. In all cases, the tests were carried out in the dark, *i.e.* without the presence of UV light which means that no photo-excited phenomena occur and consequently the biocide activity does not depend on ROS formation.

One advantage of the microparticles of the present invention is that the antibacterial and antifungal activity is attained at a very low dose, only 3 ppm or 3 mg/L, without ROS generation and low Zn²⁺ release that it highly relevant for practical applications. The ZnO dose used in the state of the art is in the range of 50-500 mg/L(A. Sirelkhatim et al. Review on Zinc Oxide Nanoparticles: Antibacterial Activity and Toxicity Mechanism. Nano-Micro Lett. (2015) 7: 219), and as a consequence 10 mg/L of ZnO is consider as low dose and preferably 3 ppm is consider a low dose too.

### Example 7. Accumulation of charge in the microparticles of the present invention and its effect on microorganisms.

Figure 9a shows a schematic representation of the microstructure of the present invention. The platelets (1) are organized in conically-shaped stacked (3) groups bound by their base along c-axis, as mentioned in Figure 1. Each platelet (1) attached to adjacent platelets generates a domain and each frontier of two platelets a domain wall (11). These domain walls create potential energy barriers for electrons, known as Schottky barriers (12). The presence of multidomains, therefore multidomain walls, and Schottky barriers are believed to generate uncompensated charges that accumulate at domain walls. This phenomenon results in an electric potential distributed by the present invention structure.

Kelvin Probe Microscopy (KPM) is a modified version of Atomic Force Microscopy (AFM). KPM consists in a non-destructive non-contact surface technique that allows imaging two-dimensional profiles of contact potential difference (VCPD) *i.e.* the difference in the work functions of the tip and the sample. For a representative sample of the microparticles of the present invention (sample D), the contact potential difference (CPD) voltage reaches values around -5 V in the top of the structure. The negative charge values were confirmed in aqueous solution of the microparticles of the present invention by using zeta potential measurements (Figure 9b). As can be seen, the present invention shows a zeta potential value around -20mV in the pH range of 6 to 10. This observation confirmed the existence of negative charge accumulation along the structure of the present invention.

The effects of the interactions between the particles of the present invention and selected microorganisms are represented in Figure 10. SEM micrographs show the damage caused by flower-like particles of the invention in contact against bacteria *E. coli* (Figure 10a) and *S*. *aureus* (Figure 10b) and fungus *A. niger* (Figure 10c). The contact is believed to be produced by electrostatic interactions between the charge of the microparticles of the present invention and the microorganisms. The main damages to microorganisms (white arrows) were abrasions such as wrinkles, flattening or blebs, cracking and/or rupture of the membrane envelope. As a result, these abnormal textures lead to "holey" membrane and finally to microorganism death.

Cathodoluminescence (CL) is an optical and electromagnetic phenomenon in which an electron beam impacts on a luminescent material causing the emission of visible light. A cathodoluminescence detector attached to a Scanning Electron Microscope (SEM), commonly termed SEM-CL, is capable of producing high-resolution digital cathodoluminescent images of luminescent materials. Figure 10d shows SEM-CL of microstructure present invention (sample D) against *A. niger* fungus. The most luminescent areas which were mostly terminal zones correspond to defects in the ZnO crystal. The areas with more defects accumulate more charge, which directly affects microorganisms, in this case *A. niger* (dark areas). As can be seen, the interaction between the flowers-like particles of the invention and microorganism through a contact mechanism does not weaken its potential charge. Therefore, the microparticles of the present invention maintained their activity for long periods of time and at very low concentrations or dose.

## Claims

1. Zinc oxide microparticles comprising platelets, wherein the platelets,
- have an edge length between 30 and 200 nm, and
- are stacked in direct contact with each other along the stacking c-axis, by means of their [0001̅] or [0001] crystalline planes, and
- are rotated by a non-zero angle with respect to their adjacent platelets along said stacking c-axis;
wherein said zinc oxide microparticles are **characterised by** a specific surface area which is less than or equal to 4 m²/g.

2. The zinc oxide microparticles according to claim 1 wherein the specific surface area is less than or equal to 2 m²/g.

3. The zinc oxide microparticles according to any of claims 1 to 2 **characterised by** being flower-like shaped microparticle structures.

4. Method for preparing the zinc oxide microparticles according to any of claims 1 to 3, comprising the steps of:
i) adding urea to an aqueous zinc salt solution,
ii) heating the solution resulting from step (i),
iii) isolating the product resulting from step (ii), and
iv) submitting the isolated product from step (iii) to an annealing treatment.

5. Method according to claim 4 wherein the zinc salt solution of step (i) is at a concentration between 1 and 10 M.

6. Method according to any of claims 4 to 5 wherein the solution in step (ii) is heated to a temperature between 80 °C and 140 °C for a period of time between 1 and 3 hours.

7. Method according to any of claims 4 to 6, wherein step (iii) comprises:
- cooling down the solution resulting from step (ii) to room temperature, and adding a hot aqueous solvent to obtain a precipitated product, and
- isolating the precipitated product.

8. Method according to any of claims 4 to 7, wherein the annealing treatment of step (iv) is performed at a temperature between 350 °C and 700 °C.

9. Method according to any of claims 4 to 8 wherein the annealing treatment of step (iv) is performed for a period of time between 1 and 24 hours.

10. A cosmetic product, paint, ink, paper, cardboard, textile, food, agricultural, home care, air conditioning, animal care, personal and work hygiene, contact lens, chromatography material, medical equipment, dermatological, lacquer, coating and/or plastic product containing the zinc oxide microparticles according to any of claims 1 to 3.

11. Biocide composition comprising the zinc oxide microparticles according to any of claims 1 to 3 and optionally a carrier.

12. Use of the biocide composition according to claim 11 for the elimination, inhibition of the growth, or inhibition of progeny of microorganisms.

13. Use of the biocide composition according to claim 12 wherein the microorganisms are selected from bacteria and fungi.
